# EUROPEAN PATENT SPECIFICATION

(11) **EP 3 148 505 B1**
(45) Date of publication and mention of the grant of the patent: **05.12.2018**
(21) Application number: 15724321.3
(22) Date of filing: 27.05.2015
(51) Int. Cl.: A61K 8/41, A61K 8/73, A61Q 19/08, A61K 8/04

(54) **COSMETIC PROCESS FOR ATTENUATING WRINKLES**
KOSMETISCHES VERFAHREN ZUR GLÄTTUNG VON FALTEN
PROCEDE COSMETIQUE POUR ATTENUER LES RIDES

(30) Priority: 28.05.2014 FR 1454825
(43) Date of publication of application: 05.04.2017
(73) Proprietor: L'OREAL, 75008 Paris (FR)
(72) Inventor: SCHULTZE, Xavier, F-93601 AULNAY-SOUS-BOIS (FR); LION, Bertrand, F-93601 AULNAY-SOUS-BOIS (FR); GREAVES, Andrew, F-77700 Magny-le-Hongre (FR); POTTER, Anne, F-92200 Neuilly-sur-Seine (FR)
(74) Representative: Kromer, Christophe
(86) International application number: PCT/EP2015/061661
(87) International publication number: WO 2015/181211

(56) References cited:
- WO-A2-2007/106738
- FR-A1- 2 955 255
- SMEDS K A ET AL: "PHOTOCROSSLINKABLE POLYSACCHARIDES FOR IN SITU HYDROGEL FORMATION", JOURNAL OF BIOMEDICAL MATERIALS RESEARCH, WILEY, NEW YORK, NY, US, vol. 54, no. 1, 1 January 2001 (2001-01-01), pages 115-121, XP008018719, ISSN: 0021-9304, DOI: 10.1002/1097-4636(200101)54:1<115::AID-JBM 14>3.0.CO;2-Q
- BURDICK JASON A ET AL: "CONTROLLED DEGRADATION AND MECHANICAL BEHAVIOR OF PHOTOPOLYMERIZED HYALURONIC ACID NETWORKS", BIOMACROMOLECULES, AMERICAN CHEMICAL SOCIETY, US, vol. 6, 1 January 2005 (2005-01-01), pages 386-391, XP008069736, ISSN: 1525-7797, DOI: 10.1021/BM049508A

## Description

The present invention relates to a skincare process, in particular a cosmetic skincare process, intended to attenuate wrinkles, comprising the application to the skin of the mixture of a composition comprising a grafted hyaluronic acid polymer and of a particular amine compound, and to the use of this mixture as a skin tensioning agent.

During the ageing process, various signs appear on the skin, which are very characteristic of this ageing, resulting in particular in a modification of skin structure and functions. The main clinical signs of skin ageing are in particular the appearance of fine lines and deep wrinkles, which increase with age.

It is known practice to treat these signs of ageing using cosmetic or dermatological compositions containing active agents capable of combating ageing, such as α-hydroxy acids, β-hydroxyacids and retinoids. These active agents act on wrinkles by eliminating dead skin cells and by accelerating the cell renewal process. However, these active agents have the drawback of only being effective for the treatment of wrinkles after a certain application time. However, it is increasingly sought to obtain a quick or immediate effect of the active agents used, rapidly resulting in smoothing out of wrinkles and fine lines and in the disappearance of the signs of fatigue.

Hyaluronic acid is known for its skin tensioning properties.

The inventors have discovered that a hyaluronic acid polymer grafted with (meth)acrylate groups combined with a particular amine compound has an improved tensioning effect on the skin and thus makes it possible to attenuate wrinkles on the skin, notably quickly or immediately after application on the skin. Furthermore, the tensioning effect obtained also exhibits good water resistance, and therefore good persistence with respect to water.

Polymers of hyaluronic acid grafted with (meth)acrylate groups are described in document WO 2007/106738 and the publications J. Burdick et al "Controlled degradation and mechanical behavior photopolymerized hyaluronic acid networks", Biomacromolecules, 2005, 6, pages 386-391; Mark Grinstaff "Photocrosslinkable polysaccharides for in situ hydrogel formation", Journal of biomedical materials research, 2001, volume 55, Issue 2, pages 115-121. They have been used to form hydrogels after crosslinking.

More specifically, a subject of the present invention is a process, in particular a cosmetic process, for caring for the skin, more particularly facial skin, in particular wrinkled skin, comprising:
either the topical application to the skin of an extemporaneous mixture of a cosmetic composition comprising a hyaluronic acid polymer grafted with (meth)acrylate groups and an amine compound ;
or the sequential application to the skin of a cosmetic composition comprising a hyaluronic acid polymer grafted with (meth)acrylate groups and of an amine compound, or of a cosmetic composition containing same,
said amine compound being chosen among ethylenediamine, 1,3-propylenediamine, 1,4-butylenediamine,
the grafted hyaluronic acid polymer having a degree of grafting ranging from 10% to 80%,
the amine compound being used according to an amine compound/grafted hyaluronic acid (meth)acrylate group mole ratio ranging from 0.1 to 10.
The process according to the invention is in particular intended for smoothing out human facial and/or body skin and/or for decreasing or effacing the signs of skin ageing, in particular for reducing or effacing wrinkles and/or fine lines on the skin.
A subject of the invention is also the use, in particular cosmetic use, as a skin, in particular wrinkled skin, tensioning agent, of a grafted hyaluronic acid polymer as previously defined, or of a cosmetic composition containing same, mixed with the amine compound as previously defined.

A subject of the invention is also a composition, in particular a cosmetic composition, obtained by mixing a cosmetic composition comprising a grafted polymer as previously defined and an amine compound as previously defined or a composition, in particular a cosmetic composition, containing same.

A subject of the invention is also a kit comprising a first composition, in particular a cosmetic composition, comprising a grafted polymer as previously defined and a second composition comprising an amine compound as previously described, or a composition, in particular a cosmetic composition, comprising same, the first and second compositions each being packaged in a distinct packaging assembly.
The composition packaging assembly is, in a known manner, any packaging that is suitable for storing cosmetic compositions (especially a bottle, tube, spray bottle or aerosol bottle).
Such a kit allows the skin treatment process according to the invention to be performed.

The term "tensioning agent" is intended to mean compounds capable of having a noticeable tensioning effect, i.e. of smoothing out the skin and immediately reducing, or even causing to disappear, the wrinkles and fine lines.
The tensioning effect may be characterized by means of an *in vitro* retraction test as described in Example 5 hereinafter.

Hyaluronic acid is a linear glycosaminoglycan composed of repeating D-glucuronic acid and N-acetyl-D-glucosamine units linked together via alternating beta-1,4 and beta-1,3 glycosidic linkages.
Preferably, the grafted hyaluronic acid polymer has a weight-average molecular weight ranging from 5000 to 1 000 000 daltons, more preferentially ranging from 10 000 to 500 000 daltons, and even more preferentially ranging from 15 000 to 350 000 daltons.
The molecular weight can be determined in particular by liquid chromatography, the eluent being 0.1 M sodium chloride and 330 mg/l of sodium azide in water, with dextran as standard, and Wyatt Optilab T-Rex refractometer and Wyatt Dawn-Heleos II light scattering detectors.

The grafted hyaluronic acid polymer has a degree of grafting with (meth)acrylate groups ranging from 10% to 80% preferably ranging from 20% to 80%, more preferably ranging from 40% to 70%, and preferentially ranging from 45% to 65%. The degree of grafting corresponds to the mole percentage of hydroxyl groups of the hyaluronic acid which are grafted with a (meth)acrylate group.
By way of example, a degree of grafting of 50% corresponds to 2 acrylate groups grafted onto the 4 hydroxyls of the repeating unit of the hyaluronic acid.
The grafting of hyaluronic acid with (meth)acrylate groups results from the presence of a (meth)acrylate ester group formed with the free hydroxyls of hyaluronic acid.

Preferably, the hyaluronic acid is grafted with acrylate groups.
The hyaluronic acid grafted with (meth)acrylate groups can be obtained by reaction of the hyaluronic acid with (meth)acrylic anhydride. The reaction is advantageously carried out in a basic aqueous medium, in particular in the presence of an organic or inorganic base such as, for example, sodium hydroxide. Preferably, the reaction is carried out at a temperature ranging from 5 to 10°C, in particular for a period of time ranging from 24 hours to 48 hours.

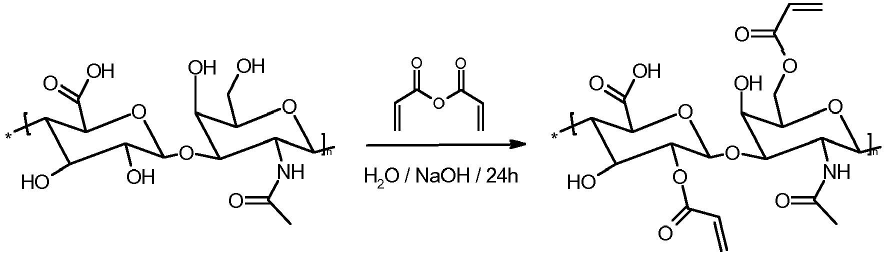

Various degrees of grafting with the (meth)acrylate groups can be obtained by varying the amount of (meth)acrylic anhydride used proportionally to the amount of hyaluronic acid.

The grafted hyaluronic acid polymer as previously defined may be present in the composition used according to the invention in a content ranging from 0.1% to 10% by weight, relative to the total weight of the composition, preferably ranging from 0.5% to 10% by weight of active material, preferentially ranging from 1% to 8% by weight, and more preferentially ranging from 1% to 6% by weight.

The amine compound according to the invention is chosen among ethylenediamine, 1,3-propylenediamine and 1,4-butylenediamine. Preferably, ethylenediamine is used.

The amine compound used in the process according to the invention is used according to an amine compound/grafted hyaluronic acid (meth)acrylate group mole ratio ranging from 0.1 to 10, preferably ranging from 0.1 to 5, and preferentially ranging from 0.1 to 2.
The amine compound in contact with the grafted hyaluronic acid reacts with the ethylenic unsaturations of the (meth)acrylate groups grafted onto the hyaluronic acid so as to form amine bonds with the end carbon of the ethylenic unsaturation of the (meth)acrylate group (this reaction is known as the Michael reaction).
Examples of this reaction are illustrated in the following schemes:

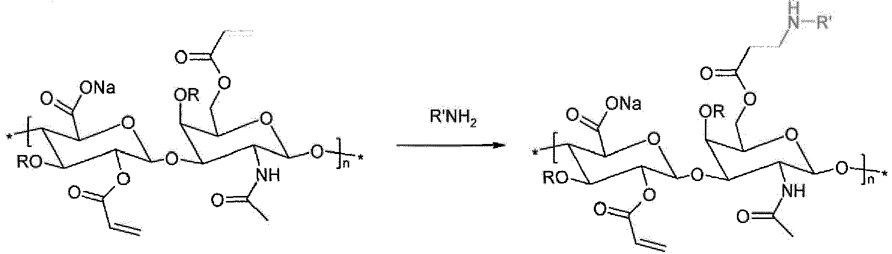

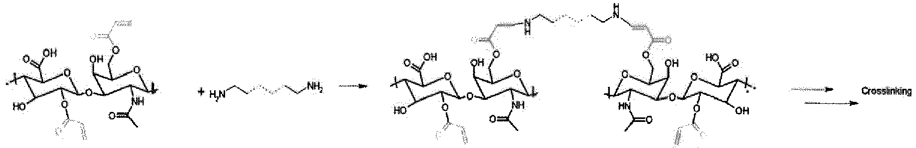

When the grafted hyaluronic acid comprises methacrylate groups, a catalyst can be used in the presence of the amine compound. This catalyst makes it possible to obtain good reactivity of the amine compound on the ethylenic unsaturation of the methacrylate group.

As an example of a catalyst, mention may be made of Lewis acids, such as boric acid, aluminium chloride or cerium chloride, and also phosphines, such as trimethylphosphine (trialkylphosphine), phenyldimethylphosphine (dialkylarylphosphine), diphenylmethylphosphine (alkyldiarylphosphine), triphenylphosphine (triarylphosphine), tricarboxyethylphosphine, and the oxide equivalents.
The composition used according to the invention is generally suitable for topical application to the skin and thus generally comprises a physiologically acceptable medium, i.e. a medium that is compatible with the skin and/or skin appendages. It is preferably a cosmetically acceptable medium, i.e. a medium which has a pleasant colour, odour and feel and which does not cause any unacceptable discomfort (stinging, tautness or redness) liable to discourage the consumer from using this composition.
The composition used according to the invention may be in any galenic form conventionally used for a topical application and especially in the form of dispersions of aqueous gel or lotion type, emulsions of liquid or semi-liquid consistency of the milk type, obtained by dispersing a fatty phase in an aqueous phase (O/W) or vice versa (W/O), or suspensions or emulsions of soft, semi-solid or solid consistency of the cream or gel type, or alternatively multiple emulsions (W/O/W or O/W/O), microemulsions, vesicular dispersions of ionic and/or nonionic type, or wax/aqueous phase dispersions. These compositions are prepared according to the usual methods. According to one preferred embodiment of the invention, the composition is in the form of an O/W emulsion or an aqueous gel.

Advantageously, the composition used according to the invention comprises water, in particular in a content which can range from 10% to 99% by weight and preferably ranging from 50% to 99% by weight, relative to the total weight of the composition.

The composition used according to the invention may also contain one or more attachments commonly used in the cosmetics field, such as emulsifiers, preservatives, sequestering agents, fragrances, thickeners, oils, waxes or film-forming polymers.

Needless to say, those skilled in the art will take care to select this or these optional additional compound(s), and/or the amount thereof, such that the anti-wrinkle properties of the composition according to the invention are not, or are not substantially, adversely affected by the envisaged addition.

According to a first embodiment of the process according to the invention, an extemporaneous mixture of a cosmetic composition comprising the (meth)acrylate-grafted hyaluronic acid polymer and of an amine compound, or a cosmetic composition containing same, as previously described, is applied to the skin. The extemporaneous mixture is advantageously prepared less than 5 minutes before it is applied to the skin, and preferably less than 3 minutes.

According to a second embodiment of the process according to the invention, the cosmetic composition comprising the (meth)acrylate-grafted hyaluronic acid polymer is first applied to the skin, then the amine compound or a cosmetic composition containing same is applied. The application of the amine compound can be carried out after a time of between 5 minutes and one hour after having applied the grafted hyaluronic acid polymer.

According to a third embodiment of the process according to the invention, the amine compound, or a cosmetic composition containing same, is first applied to the skin, then the cosmetic composition comprising the (meth)acrylate-grafted hyaluronic acid polymer is applied. The application of the grafted hyaluronic acid polymer can be carried out after a time of between 5 minutes and one hour after having applied the amine compound.

The application of the cosmetic composition used according to the invention is carried out according to the usual techniques, for example by application (in particular of creams, gels, sera or lotions) to the skin intended to be treated, in particular facial and/or neck skin, especially the skin of the area around the eyes. In the context of this process, the composition may, for example, be a care composition.

The invention will now be described with reference to the examples that follow, which are given as non-limiting illustrations.

### Example 1: Hyaluronic acid 60% functionalized with acrylic anhydride

In a thermostated reactor, 5 g of hyaluronic acid (Hyacare® 50 from Evonik) were dissolved in 100 ml of water and the mixture was maintained at a temperature of 7°C, then 14.8 g of acrylic anhydride were added dropwise over the course of approximately 2 min. The pH was adjusted to 7.7 by slowly adding (over the course of approximately one hour) sodium hydroxide at 30% in water (7 M). The mixture was left to react for 24 hours.
The mixture obtained was purified by dialysis (polymer in 150 ml of water, 3.3% by weight) on a Spectra/Por® 15 kDa membrane for 5 days in 5 litres of water (water changed 4 times, i.e. 20 litres in total), then the mixture of the purified fraction was lyophilized by freezing with a bath of dry ice + acetone at -80°C, then by placing the frozen mixture in a lyophilization apparatus for 4 days. 2.5 g of a white solid were obtained.

### Analyses:

¹H NMR D₂O: 2.45 (7.36/3) OH units functionalized for 4 OH units available.
The hyaluronic acid obtained is 60%-functionalized with acrylate groups.

### Example 2: Hyaluronic acid 50% functionalized with acrylic anhydride

The polymer was prepared according to the procedure described in Example 1, using 5 g of hyaluronic acid (Hyacare® 50 from Evonik) and 7.9 g of acrylic anhydride.
After purification, 2.5 g of a white solid were obtained.

### Analyses:

¹H NMR D₂O: 1.96 OH units functionalized for 4 OH units available.
The hyaluronic acid obtained is 50%-functionalized with acrylate groups.

### Example 3: Hyaluronic acid 18% functionalized with acrylic anhydride

The polymer was prepared according to the procedure described in Example 1, using 5 g of hyaluronic acid (Hyacare® 50 from Evonik) and 3.15 g of acrylic anhydride.
After purification, 3.2 g of a white solid were obtained.

### Analyses:

¹H NMR D₂O: 0.74 OH unit functionalized for 4 OH units.
The hyaluronic acid obtained is 18%-functionalized with acrylate groups.

### Example 4: Hyaluronic acid 12% functionalized with acrylic anhydride

The polymer was prepared according to the procedure described in Example 1, using 5 g of hyaluronic acid (Hyacare® 50 from Evonik) and 1.58 g of acrylic anhydride.
After purification, 3.3 g of a white solid were obtained.

### Analyses:

¹H NMR D₂O: 0.50 OH unit functionalized for 4 OH units.
The hyaluronic acid obtained is 12%-functionalized with acrylate groups.

### Example 5:

### Demonstration of the tensioning effect of the polymers used according to the invention

The tensioning effect of the polymers of Examples 1 to 4 mixed with ethylenediamine was evaluated by means of an *in vitro* retraction test.

This test consists in comparing, *in vitro*, the tensioning capacity of the polymer + ethylenediamine mixture to be evaluated, relative to a reference tensioning polymer: Hybridur® 875 polymer dispersion from Air Products (aqueous dispersion at 40% by weight of particles of an interpenetrated network of polyurethane and acrylic polymers). The polymer to be evaluated is deposited on a nitrile rubber strip cut from a glove sold under the reference Safeskin Nitrile Criticial No. 038846 by the company Dominique Dutscher SA, having a surface area of 3.5 cm², stretched taut beforehand on a support. An aqueous solution containing the polymer to be evaluated is therefore deposited on the elastomer strip, by depositing 1.8 mg (of solids) of polymer.
26 µl of an aqueous solution containing 7% AM of Hybridur® 875 polymer are thus deposited on a nitrile rubber strip so as to thus obtain a reference tensioning strip, and 104 µl of an aqueous solution containing 3.5% AM of acrylate-functionalized hyaluronic acid to be evaluated, premixed with the aqueous solution of ethylenediamine according to the proportions specified hereinafter, are deposited on another strip.
After drying for 24 hours at ambient temperature (25°C), the curving (retraction) of the strip treated with the grafted hyaluronic acid polymer mixed with the ethylenediamine is observed in comparison with that obtained with the control (Hybridur® 875).

The water resistance of the tensioning effect was then evaluated by immersing the rubber strips treated with the polymer to be evaluated in water at ambient temperature (25°C) for 10 minutes.

Likewise, non-modified hyaluronic acid (Hyacare® 50 from Evonik) was evaluated using an aqueous solution at 3.5% AM, and also the grafted hyaluronic acid alone (polymer of Example 1) was evaluated using an aqueous solution at 3.5% AM.

An aqueous solution containing 1% by weight of ethylenediamine was prepared.

With the polymer of Example 1:

| Example | Proportion of ethylenediamine (molar equivalent) | Amount of ethylenediamine solution (µl) | Amount of solution of acrylate-grafted hyaluronic acid of Ex 1 (µl) | Addition of water (µl) | Volume of the mixture to be taken for a strip |
|---|---|---|---|---|---|
| 1a | 1 | 46 | 104 | / | 75 |
| 1b | 0.5 | 22 | 104 | / | 63 |
| 1c | 0.25 | 11.4 | 104 | 20 | 67 |
| 1d | 0.1 | 4.6 | 104 | 20 | 64 |

The following results were obtained:

| **Polymer tested** | **Tensioning effect** | **Tension effect after immersion in water** |
|---|---|---|
| Hybridure 875 reference | correct | correct |
| Example 1a | greater than the reference | greater than the reference |
| Example 1b | greater than the reference | greater than the reference |
| Example 1c | greater than the reference | greater than the reference |
| Example 1d | greater than the reference | greater than the reference |
| Hyaluronic acid | Less than the reference | No tensioning effect |
| Polymer Example 1 | Same as reference | Less than the reference |

The results obtained show that the hyaluronic acid polymer of Example 1 mixed with ethylenediamine has a good tensioning effect, including after immersion in water. The tensioning effect obtained is greater than that of the non-modified hyaluronic acid and than that of the grafted hyaluronic acid alone.

With the polymer of Example 2:

| Example | Proportion of ethylenediamine (in molar equivalent - percentage relative to the acrylate functions available) | Amount of ethylenediamine solution (µl) | Amount of solution of acrylate-grafted hyaluronic acid of Ex 2 (µl) | Volume of the mixture to be taken for a strip |
|---|---|---|---|---|
| 2a | 1 eq - 200% | 48 | 104 | 76 |
| 2b | 0.5 eq - 100% | 24 | 104 | 64 |
| 2c | 0.38 eq - 75% | 18 | 104 | 61 |
| 2d | 0.075 eq - 15% | 3.6 | 104 | 54 |

| **Polymer tested** | **Tensioning effect** | **Tensioning effect after immersion in water** |
|---|---|---|
| Hybridure 875 reference | correct | correct |
| Example 2a | equivalent to the reference | equivalent to the reference |
| Example 2b | greater than the reference | greater than the reference |
| Example 2c | greater than the reference | greater than the reference |
| Example 2d | equivalent to the reference | equivalent to the reference |
| Hyaluronic acid | Less than the reference | No tensioning effect |

The results obtained show that the hyaluronic acid polymer of Example 2 mixed with ethylenediamine has a good tensioning effect, including after immersion in water. The tensioning effect obtained is greater than that of the non-modified hyaluronic acid.

With the polymer of Example 3:

| Example | Proportion of ethylenediamine (in molar equivalent - percentage relative to the acrylate functions available) | Amount of ethylenediamine solution (µl) | Amount of solution of acrylate-grafted hyaluronic acid of Ex 3 (µl) | Volume to be taken for a strip |
|---|---|---|---|---|
| 3a | 0.36 - 200% | 20 | 104 | 62 |
| 3b | 0.18 - 100% | 10 | 104 | 57 |
| 3c | 0.13 - 75% | 7.2 | 104 | 55.6 |
| 3d | 0.03 - 15% | 1.7 | 104 | 52.8 |

| **Polymer tested** | **Tensioning effect** | **Tensioning effect after immersion in water** |
|---|---|---|
| Hybridure 875 reference | Correct | correct |
| Example 3a | equivalent to the reference | equivalent to the reference |
| Example 3b | greater than the reference | greater than the reference |
| Example 3c | greater than the reference | greater than the reference |
| Example 3d | equivalent to the reference | equivalent to the reference |
| Hyaluronic acid | Less than the reference | No tensioning effect |

The results obtained show that the hyaluronic acid polymer of Example 3 mixed with ethylenediamine has a good tensioning effect, including after immersion in water. The tensioning effect obtained is greater than that of the non-modified hyaluronic acid.

With the polymer of Example 4:

| Example | Proportion of ethylenediamine (in molar equivalent - percentage relative to the acrylate functions available) | Amount of ethylenediamine solution (µl) | Amount of solution of acrylate-grafted hyaluronic acid of Ex 3 (µl) | Volume of the mixture to be taken for a strip |
|---|---|---|---|---|
| 4a | 0.24 - 200% | 13.6 | 104 | 58.8 |
| 4b | 0.12 - 100% | 6.8 | 104 | 55.4 |
| 4c | 0.09 - 75% | 5.1 | 104 | 54.5 |
| 4d | 0.02 - 15% | 1.1 | 104 | 52.5 |

| **Polymer tested** | **Tensioning effect** | **Tensioning effect after immersion in water** |
|---|---|---|
| Hybridure 875 reference | correct | correct |
| Example 4a | equivalent to the reference | Less than the reference |
| Example 4b | equivalent to the reference | Less than the reference |
| Example 4c | equivalent to the reference | Less than the reference |
| Example 4d | equivalent to the reference | Less than the reference |
| Hyaluronic acid | Less than the reference | No tensioning effect |

The results obtained show that the hyaluronic acid polymer of Example 4 mixed with ethylenediamine has a good tensioning effect, including after immersion in water. The tensioning effect obtained is greater than that of the non-modified hyaluronic acid.

### Example 6:

An anti-wrinkle gel having the following composition was prepared:

| | |
|---|---|
| - polymer of Example 1 | 3 g |
| - hydroxyethylcellulose (NATROSOL® 250 HHR CS from Ashland) | 0.5 g |
| - Preservatives qs | |
| -Water qs | 100 g |

A similar composition was also prepared using the polymer of Example 2 or 3 or 4.

Just before the application to the skin, 405 mg of ethylenediamine are added to the gel.

The compositions obtained, as a mixture with ethylenediamine, when they are applied to the face, make it possible to effectively smooth out wrinkles.

## Claims

1. Cosmetic process for caring for the skin, more particularly facial skin, in particular wrinkled skin, comprising:
either the topical application to the skin of an extemporaneous mixture of a cosmetic composition comprising a hyaluronic acid polymer grafted with (meth)acrylate groups and an amine compound, or a cosmetic composition containing same;
or the sequential application to the skin of a cosmetic composition comprising a hyaluronic acid polymer grafted with (meth)acrylate groups and of an amine compound, or of a cosmetic composition containing same,
said amine compound being chosen among ethylenediamine, 1,3-propylenediamine, 1,4-butylenediamine,
the grafted hyaluronic acid polymer having a degree of grafting ranging from 10% to 80%,
the amine compound being used according to an amine compound/grafted hyaluronic acid (meth)acrylate group mole ratio ranging from 0.1 to 10.

2. Process according to the preceding claim, **characterized in that** the grafted hyaluronic acid polymer has a degree of grafting ranging from 40% to 70%.

3. Process according to Claim 1 or 2, **characterized in that** the hyaluronic acid polymer is grafted with acrylate groups.

4. Process according to any one of the preceding claims, **characterized in that** the grafted hyaluronic acid polymer has a weight-average molecular weight ranging from 5000 to 1 000 000 daltons, preferably ranging from 10 000 to 500 000 daltons, preferentially ranging from 15 000 to 350 000 daltons.

5. Process according to any one of the preceding claims, **characterized in that** the hyaluronic acid polymer is present in the composition in a content ranging from 0.1% to 10% by weight, relative to the total weight of the composition, preferably from 0.5% to 10% by weight of active material, preferentially ranging from 1% to 8% by weight, and more preferentially ranging from 1% to 6% by weight.

6. Process according to any one of the preceding claims, **characterized in that** the amine compound is ethylenediamine.

7. Process according to any one of the preceding claims, **characterized in that** the amine compound is used according to an amine compound/grafted hyaluronic acid (meth)acrylate group mole ratio ranging from 0.1 to 5, preferentially ranging from 0.1 to 2.

8. Process according to any one of the preceding claims, **characterized in that** an extemporaneous mixture, prepared less than 5 minutes before the application to the skin, of the cosmetic composition comprising the (meth)acrylate-grafted hyaluronic acid polymer and of the amine composition, is applied to the skin.

9. Process according to any one of Claims 1 to 7, **characterized in that** the cosmetic composition comprising the (meth)acrylate-grafted hyaluronic acid polymer is first applied to the skin, then the amine compound or a cosmetic composition containing same is applied.

10. Process according to any one of Claims 1 à 7, **characterized in that** the amine compound, or a cosmetic composition containing same, is first applied to the skin, then the cosmetic composition comprising the (meth)acrylate-grafted hyaluronic acid polymer is applied.

11. Process according to any one of the preceding claims, **characterized in that** the composition is in the form of an O/W emulsion or an aqueous gel.

12. Process according to any one of the preceding claims, **characterized in that** it is intended for attenuating wrinkles.

13. Cosmetic use, as a skin tensioning agent, in particular a wrinkled-skin tensioning agent, of the mixture of a grafted hyaluronic acid polymer as defined in any one of Claims 1 to 4 and of an amine compound as defined in one of Claims 1 and 6.

14. Cosmetic composition obtained by mixing a cosmetic composition comprising a grafted hyaluronic acid polymer as defined in any one of Claims 1 to 4 and an amine compound as defined in one of Claims 1 and 6, or a cosmetic composition containing same.

15. Kit comprising a first cosmetic composition comprising a grafted hyaluronic acid polymer as defined in any one of Claims 1 to 4 and a second composition comprising an amine compound as defined in one of Claims 1 and 6, or a cosmetic composition containing same, the first and second compositions each being packaged in a distinct packaging assembly.

## Patentansprüche

1. Kosmetisches Verfahren zur Pflege der Haut, insbesondere der Gesichtshaut, insbesondere faltiger Haut, umfassend:
entweder die topische Applikation einer Rezepturmischung einer kosmetischen Zusammensetzung, die ein mit (Meth)acrylatgruppen gepfropftes Hyaluronsäurepolymer und eine Aminverbindung umfasst, oder einer diese enthaltenden kosmetischen Zusammensetzung auf die Haut;
oder die aufeinanderfolgende Applikation einer kosmetischen Zusammensetzung, die ein mit (Meth)acrylatgruppen gepfropftes Hyaluronsäurepolymer und eine Aminverbindung umfasst, oder einer diese enthaltenden kosmetischen Zusammensetzung auf die Haut,
wobei die Aminverbindung aus Ethylendiamin, 1,3-Propylendiamin, 1,4-Butylendiamin ausgewählt ist,
wobei das gepfropfte Hyaluronsäurepolymer einen Pfropfgrad von 10% bis 80% aufweist,
wobei die Aminverbindung entsprechend einem Molverhältnis von Aminverbindung/gepfropfter Hyaluronsäure(meth)acrylat-Gruppe im Bereich von 0,1 bis 10 verwendet wird.

2. Verfahren nach dem vorhergehenden Anspruch, **dadurch gekennzeichnet, dass** das gepfropfte Hyaluronsäurepolymer einen Pfropfgrad im Bereich von 40% bis 70% aufweist.

3. Verfahren nach Anspruch 1 oder 2, **dadurch gekennzeichnet, dass** das Hyaluronsäurepolymer mit Acrylatgruppen gepfropft ist.

4. Verfahren nach einem der vorhergehenden Ansprüche, **dadurch gekennzeichnet, dass** das gepfropfte Hyaluronsäurepolymer ein gewichtsgemitteltes Molekulargewicht im Bereich von 5000 bis 1000000 Dalton, vorzugsweise im Bereich von 10000 bis 500000 Dalton, vorzugsweise im Bereich von 15000 bis 350000 Dalton aufweist.

5. Verfahren nach einem der vorhergehenden Ansprüche, **dadurch gekennzeichnet, dass** das Hyaluronsäurepolymer in der Zusammensetzung in einem Gehalt im Bereich von 0,1 bis 10 Gew.-%, bezogen auf das Gesamtgewicht der Zusammensetzung, vorzugsweise 0,5 bis 10 Gew.-% Wirkstoff, vorzugsweise im Bereich von 1 bis 8 Gew.-% und stärker bevorzugt im Bereich von 1 bis 6 Gew.-% vorliegt.

6. Verfahren nach einem der vorhergehenden Ansprüche, **dadurch gekennzeichnet, dass** die Aminverbindung Ethylendiamin ist.

7. Verfahren nach einem der vorhergehenden Ansprüche, **dadurch gekennzeichnet, dass** die Aminverbindung gemäß einem Molverhältnis von Aminverbindung/gepfropfter Hyaluronsäure(meth)acrylat-Gruppe im Bereich von 0,1 bis 5, vorzugsweise im Bereich von 0,1 bis 2 verwendet wird.

8. Verfahren nach einem der vorhergehenden Ansprüche, **dadurch gekennzeichnet, dass** eine Rezepturmischung, die weniger als 5 Minuten vor der Applikation auf die Haut hergestellt wurde, der kosmetischen Zusammensetzung, die das (Meth)acrylat-gepfropfte Hyaluronsäurepolymer und die Aminzusammensetzung umfasst, auf die Haut aufgetragen wird.

9. Verfahren nach einem der Ansprüche 1 bis 7, **dadurch gekennzeichnet, dass** die kosmetische Zusammensetzung, die das (Meth)acrylat-gepfropfte Hyaluronsäurepolymer umfasst, zuerst auf die Haut aufgetragen wird, dann die Aminverbindung oder eine diese enthaltende kosmetische Zusammensetzung aufgetragen wird.

10. Verfahren nach einem der Ansprüche 1 bis 7, **dadurch gekennzeichnet, dass** die Aminverbindung oder eine diese enthaltende kosmetische Zusammensetzung zuerst auf die Haut aufgetragen wird, dann die kosmetische Zusammensetzung, die das (Meth)acrylat-gepfropfte Hyaluronsäurepolymer umfasst, aufgetragen wird.

11. Verfahren nach einem der vorhergehenden Ansprüche, **dadurch gekennzeichnet, dass** die Zusammensetzung in Form einer O/W-Emulsion oder eines wässrigen Gels vorliegt.

12. Verfahren nach einem der vorhergehenden Ansprüche, **dadurch gekennzeichnet, dass** es dazu vorgesehen ist, Falten zu glätten.

13. Kosmetische Verwendung des Gemischs aus einem gepfropften Hyaluronsäurepolymer nach einem der Ansprüche 1 bis 4 und einer Aminverbindung nach einem der Ansprüche 1 und 6 als Hautstraffungsmittel, insbesondere Hautstraffungsmittel für faltige Haut.

14. Kosmetische Zusammensetzung, erhalten durch Mischen einer kosmetischen Zusammensetzung, umfassend ein gepfropftes Hyaluronsäurepolymer nach einem der Ansprüche 1 bis 4, und eine Aminverbindung nach einem der Ansprüche 1 und 6, oder eine diese enthaltende kosmetische Zusammensetzung.

15. Kit, umfassend eine erste kosmetische Zusammensetzung, umfassend ein gepfropftes Hyaluronsäurepolymer nach einem der Ansprüche 1 bis 4, und eine zweite Zusammensetzung, umfassend eine Aminverbindung nach einem der Ansprüche 1 und 6, oder eine diese enthaltende kosmetische Zusammensetzung, wobei die erste und zweite Zusammensetzungen jeweils in einer getrennten Verpackungseinheit verpackt sind.

## Revendications

1. Procédé cosmétique de soin de la peau, plus particulièrement de la peau du visage, en particulier de la peau ridée, comprenant :
soit l'application topique sur la peau d'un mélange extemporané d'une composition cosmétique comprenant un polymère d'acide hyaluronique greffé de groupements (méth)acrylate et d'un composé aminé, ou d'une composition cosmétique le contenant ;
soit l'application séquentielle sur la peau d'une composition cosmétique comprenant un polymère d'acide hyaluronique greffé de groupements (méth)acrylate et d'un composé aminé, ou d'une composition cosmétique le contenant,
ledit composé aminé étant choisi parmi l'éthylènediamine, la 1,3-propylènediamine, la 1,4-butylènediamine,
le polymère d'acide hyaluronique greffé ayant un taux de greffage allant de 10 % à 80 %,
le composé aminé étant utilisé selon un ratio molaire composé aminé/groupe (méth)acrylate de l'acide hyaluronique greffé allant de 0,1 à 10.

2. Procédé selon la revendication précédente, **caractérisé en ce que** le polymère d'acide hyaluronique greffé a un taux de greffage allant de 40 % à 70 %.

3. Procédé selon la revendication 1 ou 2, **caractérisé en ce que** le polymère d'acide hyaluronique est greffé de groupements acrylate.

4. Procédé selon l'une quelconque des revendications précédentes, **caractérisé en ce que** le polymère d'acide hyaluronique greffé a un poids moléculaire moyen en poids allant de 5 000 à 1 000 000 daltons, de préférence allant de 10 000 à 500 000 daltons, préférentiellement allant de 15 000 à 350 000 daltons.

5. Procédé selon l'une quelconque des revendications précédentes, **caractérisé en ce que** le polymère d'acide hyaluronique est présent dans la composition en une teneur allant de 0,1 % à 10 % en poids, par rapport au poids total de la composition, de préférence de 0,5 % à 10 % en poids de matière active, et préférentiellement allant de 1 % à 8 % en poids, et plus préférentiellement allant de 1 % à 6 % en poids.

6. Procédé selon l'une quelconque des revendications précédentes, **caractérisé en ce que** le composé aminé est l'éthylènediamine.

7. Procédé selon l'une quelconque des revendications précédentes, **caractérisé en ce que** le composé aminé est utilisé selon un ratio molaire composé aminé/groupe (méth)acrylate de l'acide hyaluronique greffé allant de 0,1 à 5, et préférentiellement allant de 0,1 à 2.

8. Procédé selon l'une quelconque des revendications précédentes, **caractérisé en ce que** l'on applique sur la peau un mélange extemporané préparé moins de 5 minutes avant l'application sur la peau de la composition cosmétique comprenant le polymère d'acide hyaluronique greffé (méth)acrylate et du composé aminé.

9. Procédé selon l'une quelconque des revendications 1 à 7, **caractérisé en ce que** l'on applique sur la peau d'abord la composition cosmétique comprenant le polymère d'acide hyaluronique greffé (méth)acrylate puis l'on applique le composé aminé, ou une composition cosmétique le contenant.

10. Procédé selon l'une quelconque des revendications 1 à 7, **caractérisé en ce que** l'on applique sur la peau d'abord le composé aminé, ou une composition cosmétique le contenant, puis l'on applique la composition cosmétique comprenant le polymère d'acide hyaluronique greffé (méth)acrylate.

11. Procédé selon l'une quelconque des revendications précédentes, **caractérisé en ce que** la composition se présente sous forme d'une émulsion H/E ou d'un gel aqueux.

12. Procédé selon l'une quelconque des revendications précédentes, **caractérisé en ce qu'**il est destiné à atténuer les rides.

13. Utilisation cosmétique en tant qu' agent tenseur de la peau, en particulier un agent tenseur d'une peau ridée, du mélange d'un polymère d'acide hyaluronique greffé tel que défini dans l'une quelconque des revendications 1 à 4 et d'un composé aminé tel que défini dans l'une des revendications 1 à 6.

14. Composition cosmétique obtenue par mélange d'une composition cosmétique comprenant un polymère d'acide hyaluronique greffé tel que défini dans l'une quelconque des revendications 1 à 4 et d'un composé aminé tel que défini dans l'une des revendications 1 et 6, ou d'une composition cosmétique le contenant.

15. Kit comprenant une première composition cosmétique comprenant un polymère d'acide hyaluronique greffé tel que défini dans l'une quelconque des revendications 1 à 4 et une deuxième composition comprenant un composé aminé tel que défini dans l'une des revendications 1 et 6, ou d'une composition cosmétique le contenant, les première et deuxième compositions étant conditionnées chacune dans un ensemble de conditionnement distinct.
